# EUROPEAN PATENT SPECIFICATION

(11) **EP 4 294 467 B1**
(45) Date of publication and mention of the grant of the patent: **18.11.2026**
(21) Application number: 22756776.5
(22) Date of filing: 15.02.2022
(51) Int. Cl.: A61L 15/28, A61L 31/10, A61L 31/14, A61L 31/04

(54) **POLYSACCHARIDE-GLYCEROL PENETRATION-RESISTANT COMPOSITIONS AND SURGICAL BARRIERS MADE THEREFROM**
PENETRATIONSRESISTENTE POLYSACCHARID-GLYCEROL-ZUSAMMENSETZUNGEN UND DARAUS HERGESTELLTE CHIRURGISCHE BARRIEREN
COMPOSITIONS DE POLYSACCHARIDE-GLYCÉROL RÉSISTANTES À LA PÉNÉTRATION ET BARRIÈRES CHIRURGICALES FABRIQUÉES À PARTIR DE CELLES-CI

(30) Priority: 16.02.2021 US 202163149886 P
(43) Date of publication of application: 27.12.2023
(73) Proprietor: Cornell University, Ithaca, New York 14850 (US)
(72) Inventor: PUTNAM, David, Ithaca, New York 14850 (US)
(74) Representative: Reddie & Grose LLP
(86) International application number: PCT/US2022/016425
(87) International publication number: WO 2022/177891

(56) References cited:
- EP-B1- 0 815 879
- WO-A1-2020/226587
- WO-A2-2020/089883
- US-A1- 2009 318 843
- US-A1- 2012 039 959
- US-A1- 2012 082 712
- US-A1- 2013 052 236

## Description

### CROSS REFERENCE TO RELATED APPLICATIONS

This application claims the benefit of priority from U.S. Provisional Application No. 63/149,886, filed on February 16, 2021.

### FIELD OF THE INVENTION

The present invention generally relates to surgical barrier devices, and more particularly, barrier devices that possess suitable penetration resistance for resisting needle puncture and at the same time have the ability to dissolve into bodily tissue over a desired period of time.

### BACKGROUND OF THE INVENTION

Laparotomy, or surgical entry into the peritoneal cavity for abdominal surgery, is one of the most common surgical procedures performed in the United States with an estimated 4 million cases performed annually, with millions more performed worldwide. Closure of the peritoneal cavity after abdominal surgery requires careful re-approximation of the fascia (the strength layer of the abdominal wall) to minimize the risk of incisional hernia. Injury to the bowel during fascial closure, and the associated morbidity or mortality, may occur as a result of insufficient visualization during closure, leading to either direct needle puncture of the bowel or strangulation by suture as it is tightened out of the view of the surgeon. Currently, intraoperative maneuvers used to prevent visceral injury include use of a metal malleable retractor or the PVC Glassman Visceral Retainer to displace and shield the bowel. However, these strategies are only partially effective as neither device completely shields the viscera. More problematically, they must be removed from the peritoneal cavity prior to closure of the final few centimeters of fascia, leaving the bowel unprotected and vulnerable to injury during this most crucial phase of the operation. Inadequate visualization and protection of the bowel further contribute to increased rates of hernia recurrence as surgeons may incorporate suboptimal fascial "bites" to decrease the risk of bowel injury during closure. The mass of bowel is also typically wider than the retractor, which leads to ineffective displacement.

Another commonly used instrument in abdominal surgery is the Glassman Visceral Retractor or "FISH". This flexible device is used to shield the bowel from inadvertent injury and is quite popular. However, because the "FISH" device is made of plastic, it must be removed from the peritoneal cavity prior to tying the final several sutures, leading to "blind" suture tying, which often results in bowel loops becoming ensnared. Further, the device is often not wide enough to prevent bowel from entering the surgical field, a design flaw resulting from the need to keep it thin enough so that it can be removed from the peritoneal cavity through a relatively small opening prior to tying of the last several fascial sutures. Ultimately, the major drawback to both the current malleable retractor and "FISH," respectively, is the inherent risk they pose as retained instruments during abdominal surgery, which leads to significant postoperative morbidities, including bowel obstruction, perforation, sepsis, reoperations, and even death. In fact, retained surgical instruments are exceedingly common with an incidence between 0.3 and 1.0 per 1,000 abdominal operations despite their avoidable nature (e.g., Stawicki, S.P., et al., Retained surgical foreign bodies: A comprehensive review of risks and preventive strategies, Scand. J. Surg. 2009, 98, 8-17).

Furthermore, beyond the difficulty posed by fascial closure, post-operative bowel adhesions (the pathologic fibrotic bands that commonly develop after surgical manipulation), are a significant contributor to patient morbidity and mortality. To enumerate, abdominal post-operative adhesions occur in an alarming 90% of abdominal surgery patients, and are a major cause of bowel obstruction, bowel perforation, chronic pelvic pain, and infertility. Medical complications from abdominal adhesions are extraordinarily high with between 30% and 75% of abdominal surgery patients requiring secondary surgery to correct conditions directly related to adhesion formation, with the economic cost of abdominal tissue adhesions and their treatment exceeding $2.1 billion annually in the United States alone (e.g., Ellis, H., et al., Adhesion-related hospital readmissions after abdominal and pelvic surgery: a retrospective cohort study, Lancet. 1999, 353, 1476-1480; Ray, N.F. et al., Abdominal adhesiolysis: inpatient care and expenditures in the United States in 1994. J Am Coll Surg. 1998, 186, 1-9). Given the above limitations of surgical instruments currently employed in abdominal surgery, there would a significant benefit in a surgical barrier that could reduce the complications associated with abdominal surgery and to better facilitate fascial closure.

WO2020/089883A2 describes an oxidized cellulose composition comprising glycerol and in paste form, for use as an adhesion prevention material. US2012/039959A1 describes mono- and bi-layer alginate post-surgical adhesion barriers. WO2020/226587A1 describes a biocompatible, biodegradable and bioresorbable adhesion membrane containing hyaluronic acid, chitosan and carboxymethyl cellulose.

### SUMMARY OF THE INVENTION

In a first aspect, the present invention is directed to a resorbable surgical barrier sheet comprising:
water-soluble polysaccharide having a molecular weight of 30,000 g/mol to 500,000 g/mol;
glycerol; and
water,
wherein the water-soluble polysaccharide by weight and glycerol by volume are present at a ratio of about 1:0.8 to 1:1.2,
wherein water is present at 8-20 wt%, and
wherein the resorbable surgical barrier sheet possesses a needle penetration resistance of at least 1 Newton and an elastic modulus of 0.5-2 MPa.

In a second aspect, the present invention is directed to a resorbable surgical barrier sheet as defined herein for use in a method of preventing injury to tissue during a surgical procedure, wherein the method comprises placing the resorbable surgical barrier sheet at a surgical site.

Further features of the present invention are as defined in the appended claims.

The present disclosure is directed to a surgical barrier device having substantial flexibility yet sufficient strength and toughness to resist needle puncture, along with the advantageous ability to dissolve in bodily tissue at the surgical site after use. The surgical barrier device has the further advantage of being composed of non-toxic substances that have insignificant to no adverse effect in the human body when absorbed during the dissolution period.

The surgical barrier compositions described herein possess several advantages: 1) the capacity to be processed into thin sheets or wafers with sufficient flexibility; 2) ability to prevent or reduce inadvertent needle puncture; and 3) a rapid dissolution profile in aqueous (typically, extracellular fluid) environments, such as the intraperitoneal cavity, e.g., a 96% degradation after 4 hours when in contact with bodily fluid. Another feature is that the surgical barrier composition is reversibly adherable to biological tissue and may permit a user to remove and replace the barrier upon initial placement of the barrier or during the surgical procedure. Given these properties, these surgical barrier compositions can be utilized across multiple surgical disciplines, including as rapidly dissolving surgical shields to protect the bowel during laparotomy closure and potentially mitigate formation of post-operative bowel adhesions. These surgical barrier compositions can also be used in other surgical settings beyond abdominal or laparotomy surgery.

The surgical barrier composition is advantageously flexible and elastomeric, yet of sufficient strength to block a needle puncture to underlying tissue. The surgical barrier composition is also advantageously biocompatible and non-toxic, thereby permitting the composition, when used as a shield, to naturally dissolve and clear through the body with no adverse effect. By virtue of the biocompatible property of the surgical barrier composition, the surgeon can advantageously dispense with a post-operative procedure of extracting the surgical barrier device.

More particularly, the surgical barrier is or includes a solid flexible material composed of at least (or solely) a water-soluble polysaccharide, glycerol, and water. In embodiments, the solid flexible material is an interpenetrating polymer network (IPN) of the water-soluble polysaccharide and glycerol. In some embodiments, the water-soluble polysaccharide is a cellulose. In particular embodiments, the cellulose is methyl cellulose, carboxymethylcellulose or a salt thereof (CMC), hyaluronic acid (HA) or a combination thereof.

The water-soluble polysaccharide by weight and glycerol by volume are present in the surgical barrier composition (i.e., solid flexible material) at a ratio of about 1:0.8 to 1:1.2. In embodiments, the water-soluble polysaccharide by weight and glycerol by volume, are present at a ratio of about 1:0.8 to 1:1.15. In embodiments, the water-soluble polysaccharide by weight and glycerol by volume, are present at a ratio of about 1.1 to 1:1.15. In embodiments, the water-soluble polysaccharide by weight and glycerol by volume, are present at a ratio of about 1:1 to 1:1.2. The water is present at 8-20 wt%. In embodiments, the water is present at 10-18 wt%. In embodiments, the water is present at 12-16 wt%. In embodiments, the water is present at 14 wt%. In embodiments, the water present is determined by thermogravimetric analysis. In embodiments, the water present is determined by thermogravimetric analysis as measured between 60-140°C.

In any of the ratio embodiments described above, the water-soluble polysaccharide (such as any of those provided above) has a molecular weight of 30,000 g/mol to 500,000 g/mol. In any of the ratio embodiments described above, the water-soluble polysaccharide (such as any of those provided above) may have a molecular weight of 40,000 g/mol to 500,000 g/mol. In any of the ratio embodiments described above, the water-soluble polysaccharide (such as any of those provided above) may have a molecular weight of 30,000 g/mol to 250,000 g/mol. In any of the ratio embodiments described above, the water-soluble polysaccharide (such as any of those provided above) may have a molecular weight of 30,000 g/mol to 150,000 g/mol. In any of the ratio embodiments described above, the water-soluble polysaccharide (such as any of those provided above) may have a molecular weight of 40,000 g/mol to 150,000 g/mol. In any of the ratio embodiments described above, the water-soluble polysaccharide (such as any of those provided above) may have a molecular weight of 49,000 g/mol, 90,500 g/mol, or 250,000 g/mol. In any of the ratio embodiments described above, the water-soluble polysaccharide (such as any of those provided above) may have a molecular weight of 90,500 g/mol.

For any of the embodiments provided above, the surgical barrier has an elastic modulus of 0.5-2 MPa, for example 0.5-1.5 MPa, 0.5-1 MPa, 0.8-2 MPa, 0.8-1.5 MPa, 0.8-1 MPa, 1-2 MPa, 1-1.5 MPa, 1.25-2 MPa, or 1.5-2 MPa. For any of the embodiments provided above, the surgical barrier may have a thickness of at least 0.5 mm and up to 5 mm. For any of the embodiments provided above, the surgical barrier may have a thickness of at least 1 mm and up to 5 mm. For any of the embodiments provided above, the surgical barrier may have a thickness of at least 1 mm and up to 3 mm. For any of the embodiments provided above, the surgical barrier may have a thickness of at least 0.8 mm and up to 2 mm. For any of the embodiments provided above, the surgical barrier may have a thickness of at least 1 mm and up to 1.5 mm. In some embodiments, the surgical barrier has a non-uniform thickness. In other embodiments, the surgical barrier has a uniform thickness.

In embodiments, the surgical barrier further includes at least one component or other feature that prevents migration of the surgical barrier in the body. In embodiments, the at least one feature that prevents migration includes at least one protrusion, at least one texture, or a combination thereof. For any of the above embodiments, the surgical barrier, when placed at a surgical site, substantially dissolves within 72 hours. For any of the above embodiments, the surgical barrier, when placed at a surgical site, substantially dissolves within 24 hours. For any of the above embodiments, the surgical barrier, when placed at a surgical site, substantially dissolves within 7 hours. For any of the above embodiments, the surgical barrier, when placed at a surgical site, substantially dissolves within 3 hours. For any of the above embodiments, the surgical barrier, when placed at a surgical site, substantially dissolves within 1 hour. In embodiments, the barrier is reversibly adherable to biological tissue. For any of the above embodiments, the surgical barrier, when placed at a surgical site is reversibly adherable to biological tissue. For any of the embodiments provided above, the barrier may have a penetration resistance of at least or above 1, 1.5, or 2 Newtons.

The present application also describes a non-claimed method of preventing injury to tissue during a surgical procedure by placing a surgical barrier, such as any of the surgical barrier compositions and embodiments described above, at a surgical site. In embodiments, the water-soluble polysaccharide is a cellulose. In embodiments, the cellulose is methyl cellulose, carboxymethylcellulose or a salt thereof (CMC), hyaluronic acid (HA) or a combination thereof. In embodiments, the surgical barrier, after being placed at the surgical site, has a rate of dissolution in the first 30 minutes that is slower than the rate of dissolution after the first 30 minutes. In embodiments, the surgical barrier has a penetration resistance of up to 10 Newtons. In embodiments, the surgical barrier has a thickness of at least 0.5 mm and up to 5 mm. In embodiments, the surgical barrier has a thickness of at least 1 mm and up to 5 mm. In embodiments, the surgical barrier has a thickness of at least 1 mm and up to 3 mm. In embodiments, the surgical barrier has a thickness of at least 0.8 mm and up to 2 mm. In embodiments, the surgical barrier has a thickness of at least 1 mm and up to 1.5 mm. In embodiments, the surgical barrier has a non-uniform thickness. In embodiments, the surgical barrier has a uniform thickness. In embodiments, the surgical barrier substantially dissolves within 24 hours after being placed at the surgical site. In embodiments, the surgical barrier substantially dissolves within 7 hours after being placed at the surgical site. In embodiments, the surgical barrier substantially dissolves within 3 hours after being placed at the surgical site. In embodiments, the surgical barrier substantially dissolves within 1 hour after being placed at the surgical site.

In embodiments, the water-soluble polysaccharide by weight and glycerol by volume, are present in the surgical barrier composition (i.e., solid flexible material) at a ratio of about 1:0.8 to 1:1.2. In embodiments, the water-soluble polysaccharide by weight and glycerol by volume, are present at a ratio of about 1:0.8 to 1:1.15. In embodiments, the water-soluble polysaccharide by weight and glycerol by volume, are present at a ratio of about 1.1 to 1:1.15. In embodiments, the water-soluble polysaccharide by weight and glycerol by volume, are present at a ratio of about 1:1 to 1:1.2. In embodiments, the water is present at 8-20 wt%. In embodiments, the water is present at 10-18 wt%. In embodiments, the water is present at 12-16 wt%. In embodiments, the water is present at 14 wt%. In embodiments, the water present is determined by thermogravimetric analysis. In embodiments, the water present is determined by thermogravimetric analysis as measured between 60-140°C.

In any of the ratio embodiments of the methods described above, the water-soluble polysaccharide (such as any of those provided above) may have a molecular weight of 30,000 g/mol to 500,000 g/mol. In any of the ratio embodiments of the methods described above, the water-soluble polysaccharide (such as any of those provided above) may have a molecular weight of 40,000 g/mol to 500,000 g/mol. In any of the ratio embodiments of the methods described above, the water-soluble polysaccharide (such as any of those provided above) may have a molecular weight of 30,000 g/mol to 250,000 g/mol. In any of the ratio embodiments of the methods described above, the water-soluble polysaccharide (such as any of those provided above) may have a molecular weight of 30,000 g/mol to 150,000 g/mol. In any of the ratio embodiments of the methods described above, the water-soluble polysaccharide (such as any of those provided above) may have a molecular weight of 40,000 g/mol to 150,000 g/mol. In any of the ratio embodiments of the methods described above, the water-soluble polysaccharide (such as any of those provided above) may have a molecular weight of 49,000 g/mol, 90,500 g/mol, or 250,000 g/mol. In any of the ratio embodiments of the methods described above, the water-soluble polysaccharide (such as any of those provided above) may have a molecular weight of 90,500 g/mol.

### BRIEF DESCRIPTION OF THE FIGURES

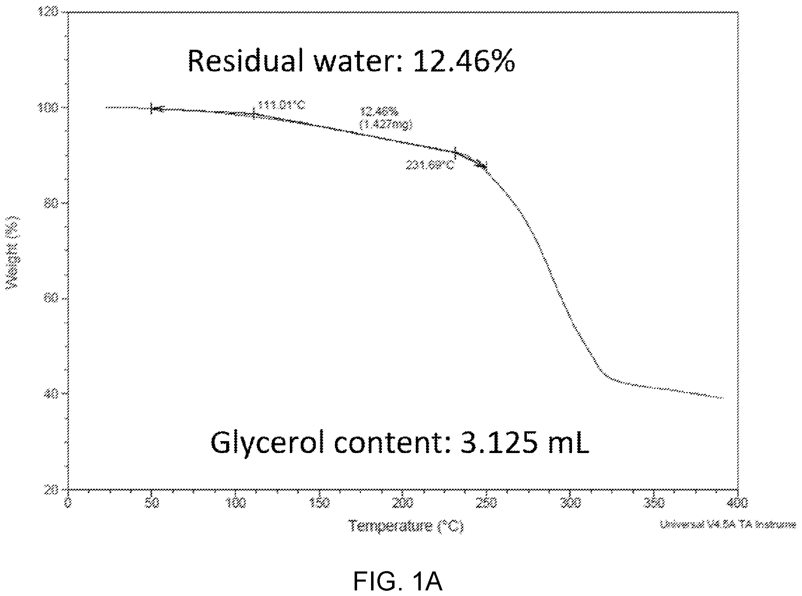

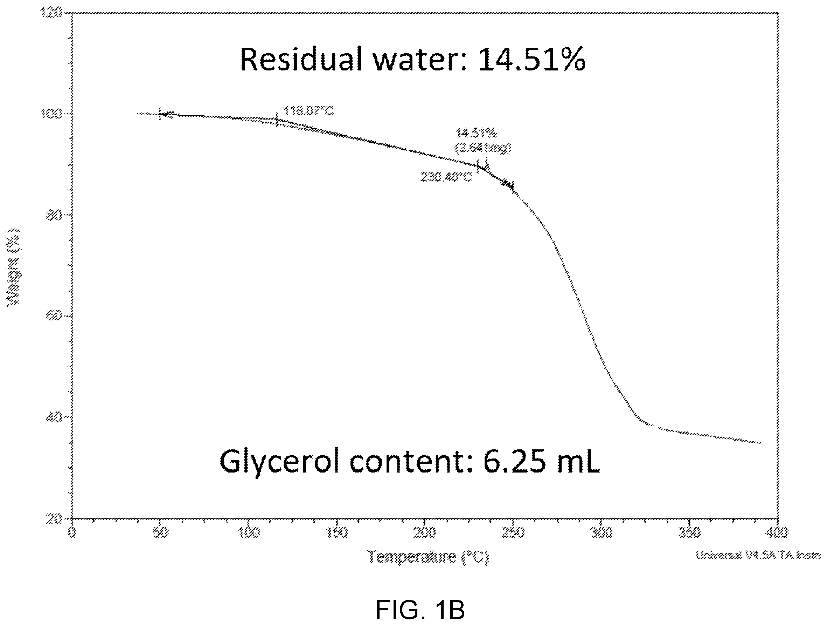

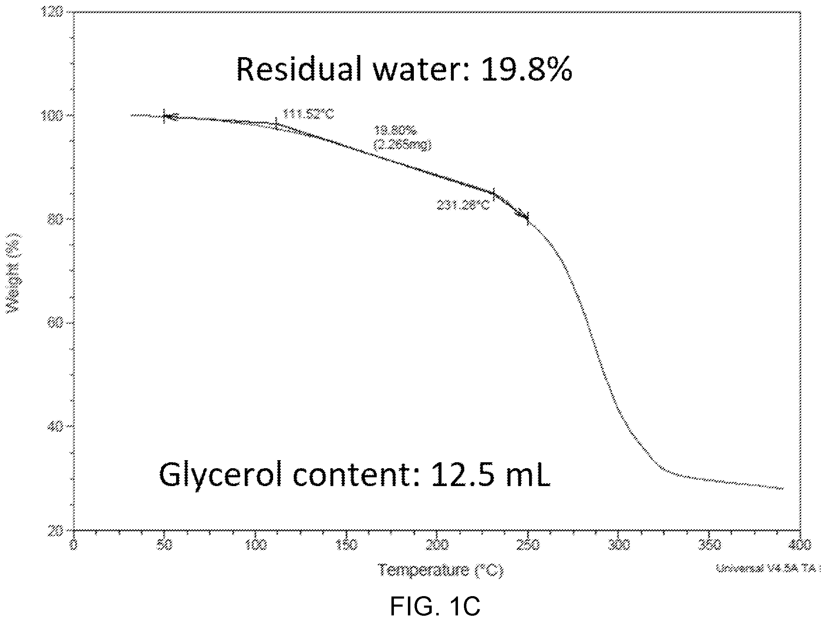

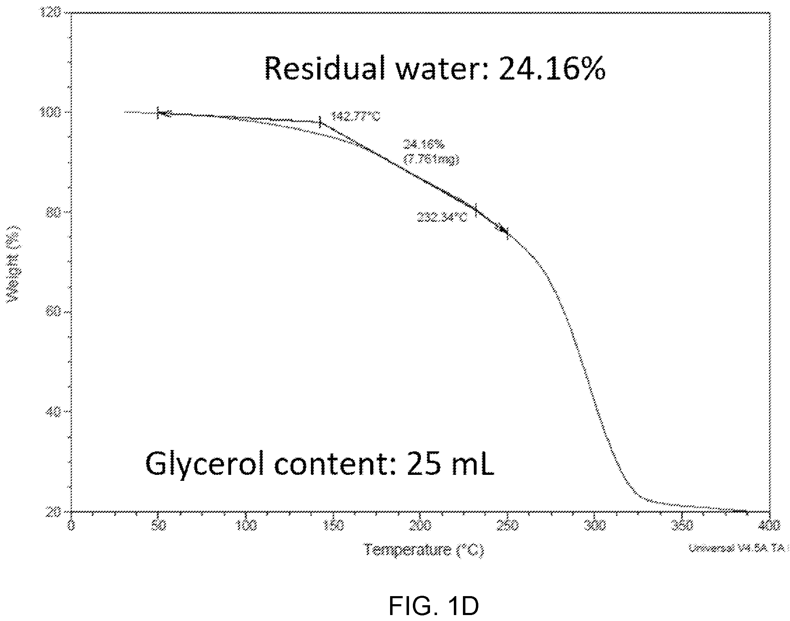

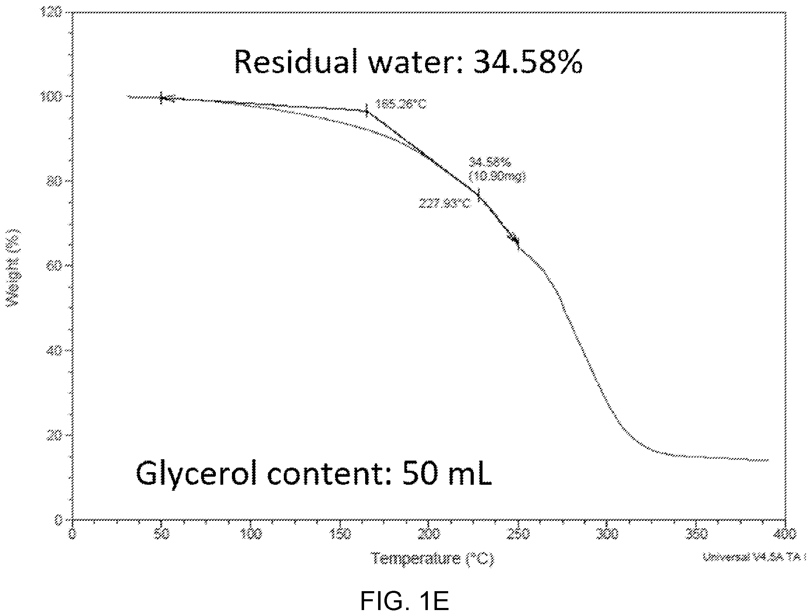

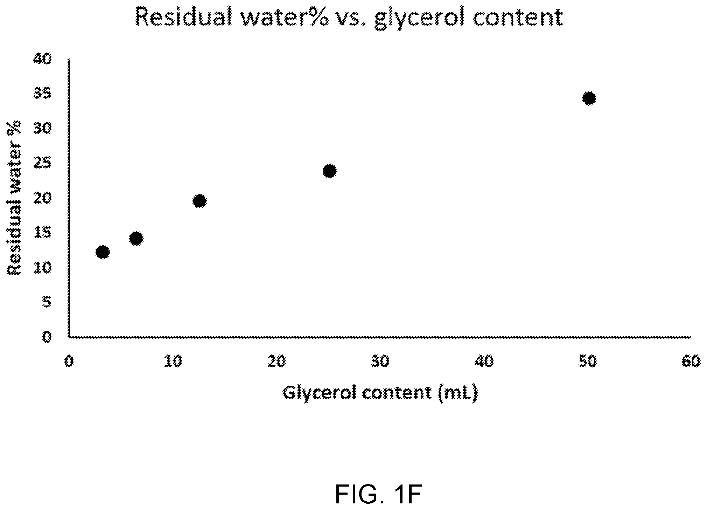

FIGS 1A-1F. FIG. 1A-1E show residual water content for each of the following surgical barrier samples: 3.125 mL glycerol content (Sample A, FIG. 1A), 6.25 mL glycerol content (Sample B, FIG. 1B), 12.5 mL glycerol content (Sample C, FIG. 1C), 25 mL glycerol content (Sample D, FIG. 1D) or 50 mL glycerol content (Sample E, FIG. 1E), as determined by thermogravimetric analysis. The residual water content is plotted against glycerol content in FIG. 1F.

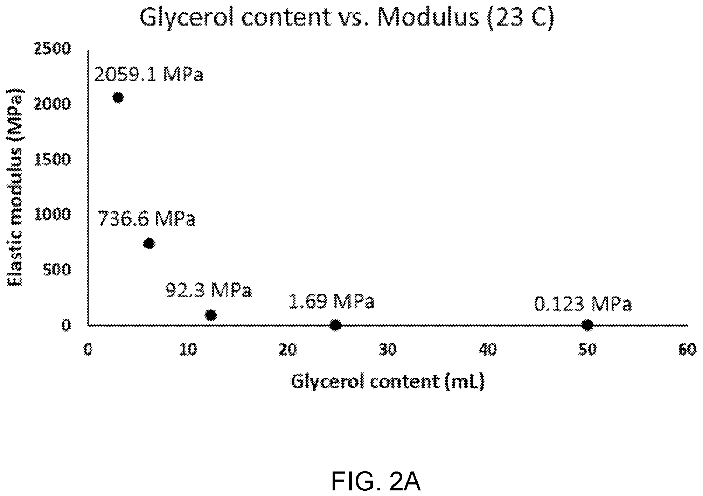

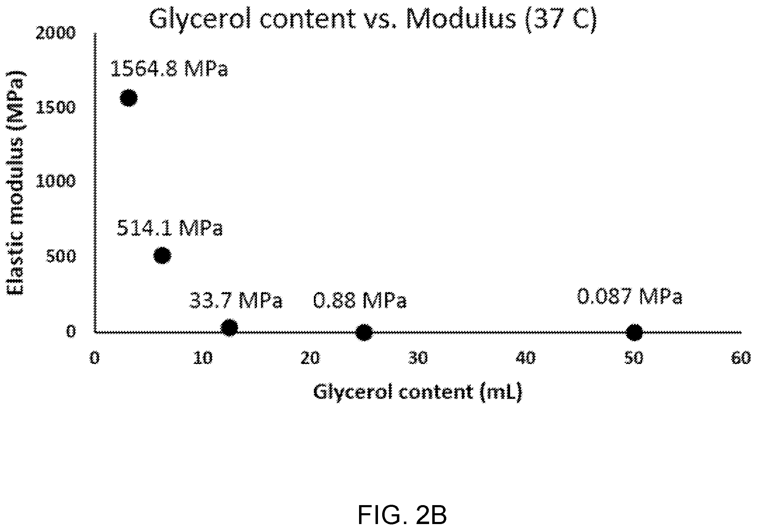

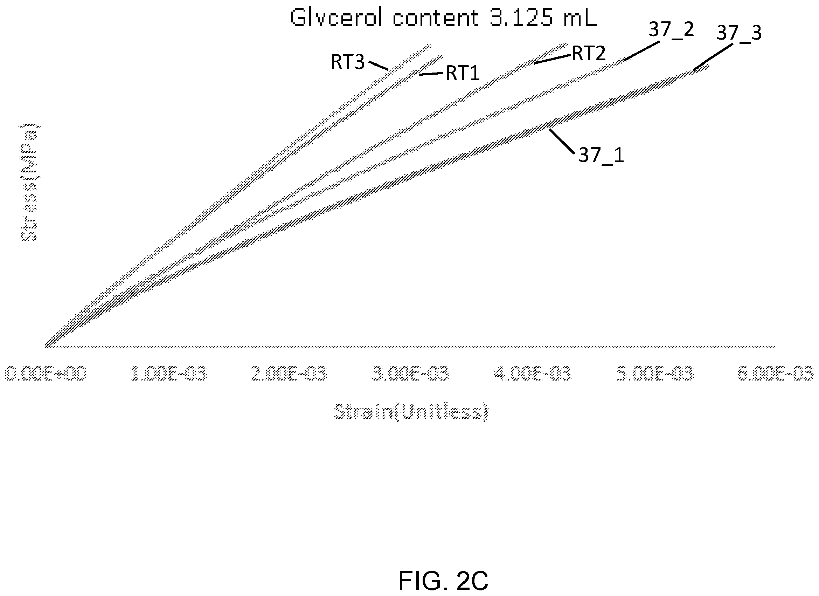

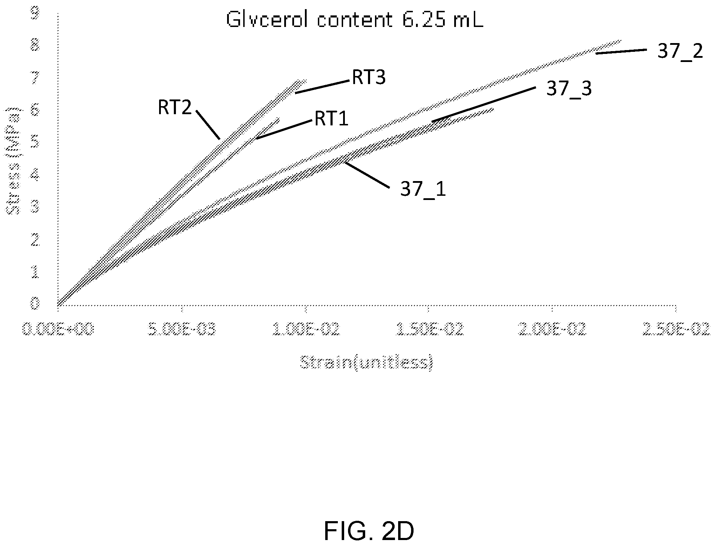

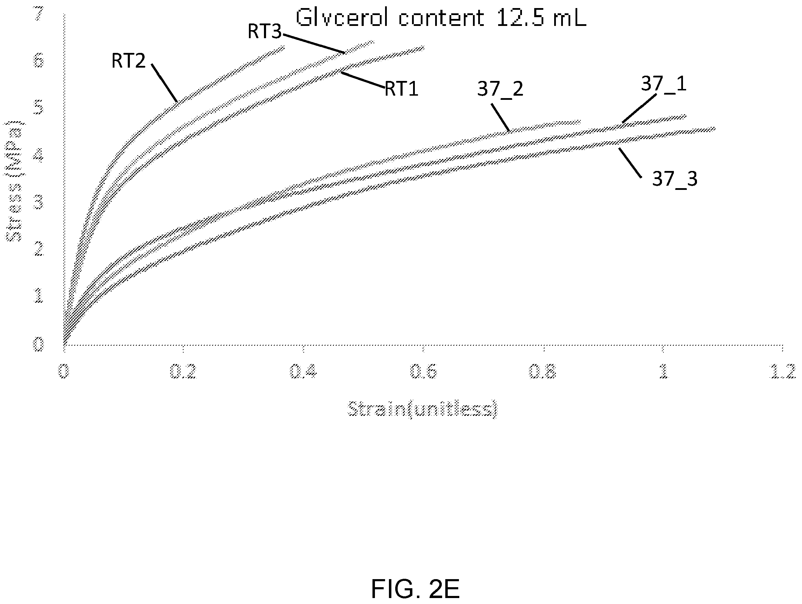

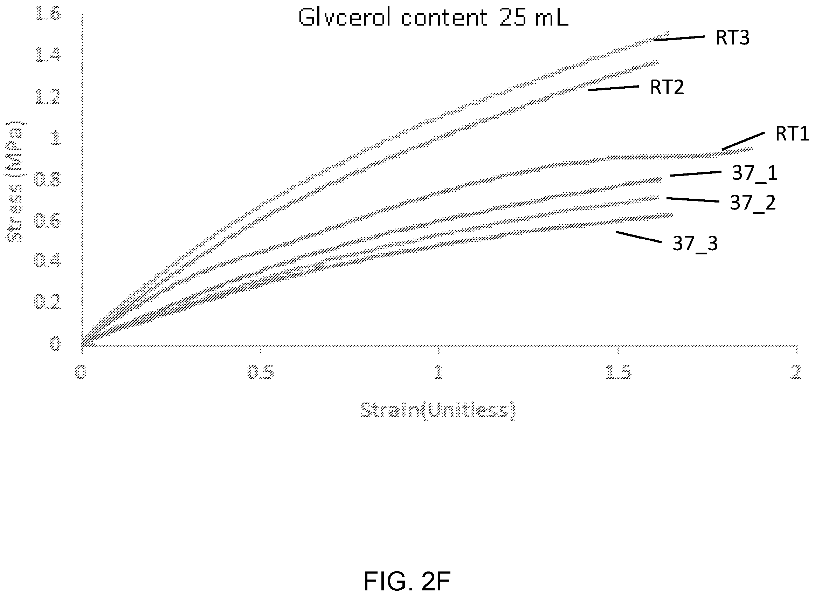

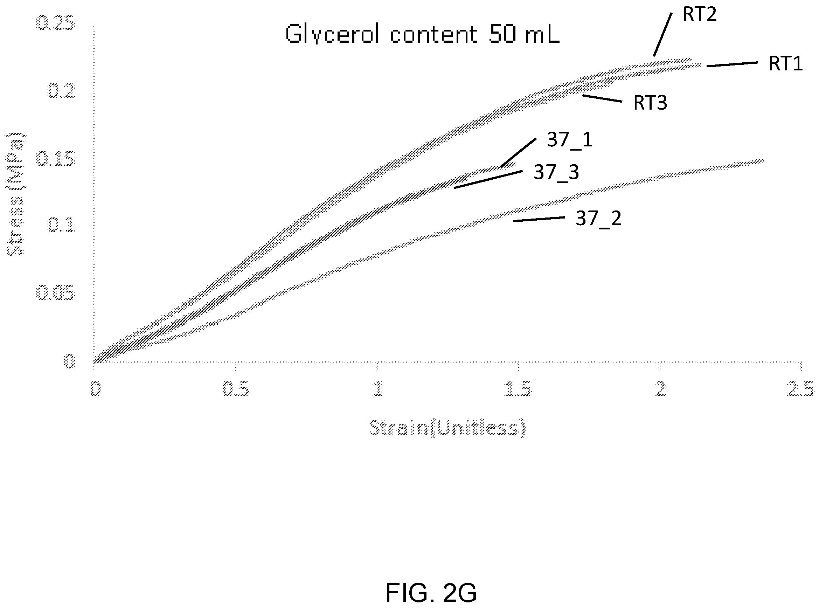

FIGS. 2A-2G. These figures show results of dynamic mechanical analysis testing of the surgical barrier compositions. FIGS. 2A and 2B show elastic modulus as a function of glycerol content at a temperature of 23°C and 37°C, respectively, for the five samples (Samples A-E) differing in glycerol content. FIGS. 2C-2G show stress-strain curves for each of the following samples: 3.125 mL glycerol content (Sample A, FIG. 2C), 6.25 mL glycerol content (Sample B, FIG. 2D), 12.5 mL glycerol content (Sample C, FIG. 2E), 25 mL glycerol content (Sample D, FIG. 2F) or 50 mL glycerol content (Sample E, FIG. 2G).

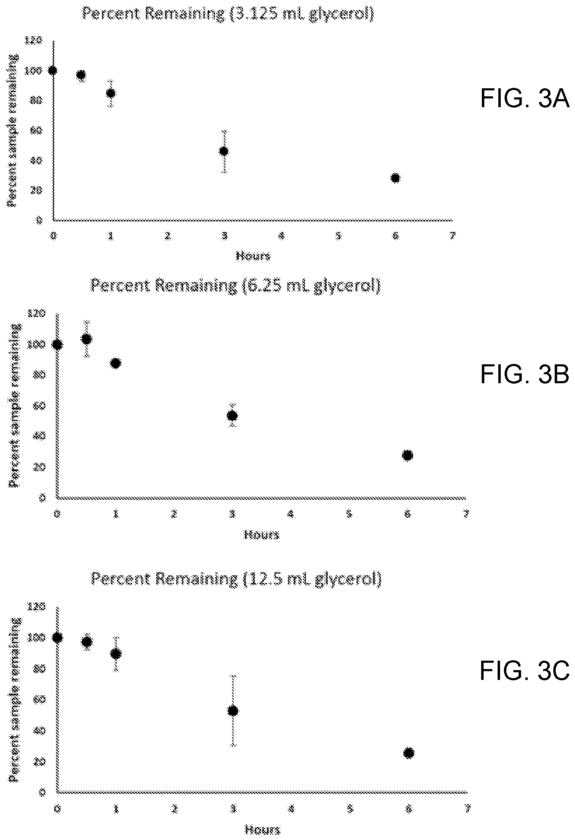

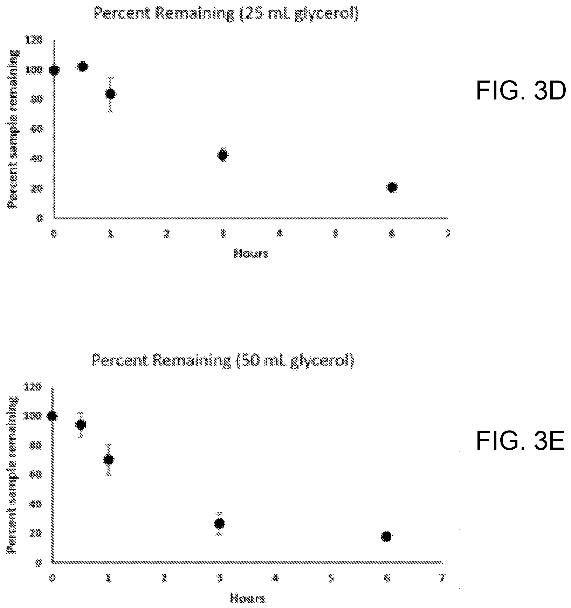

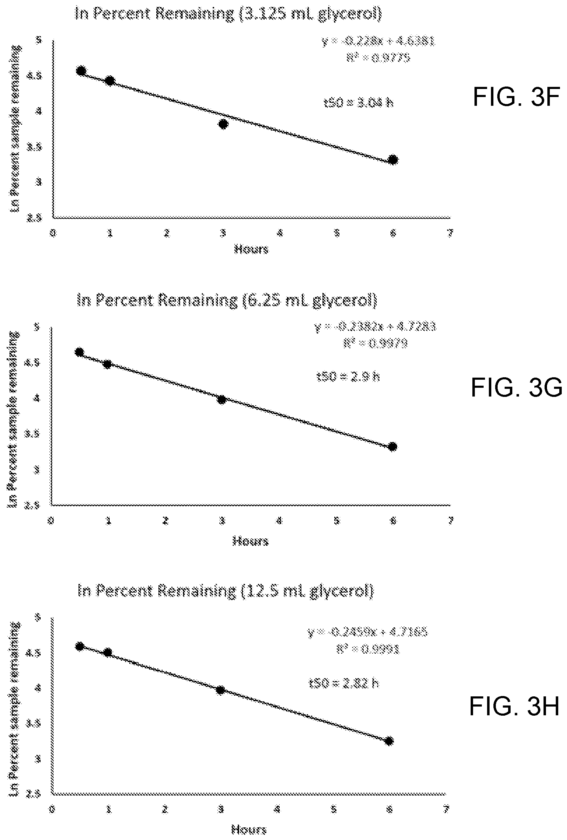

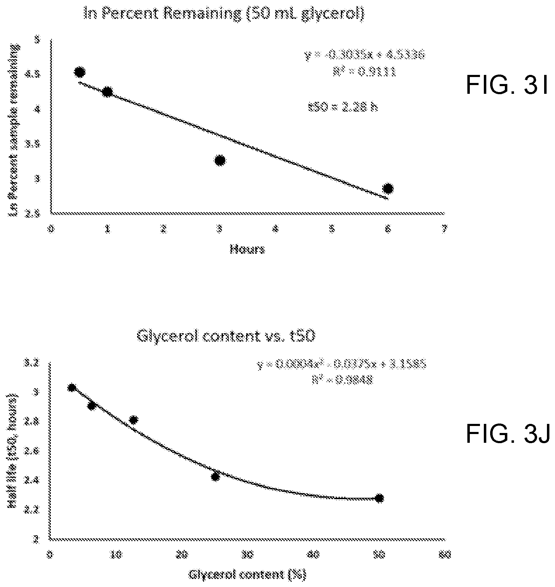

FIGS. 3A-3J. These figures show results of dissolution kinetics analysis testing of the surgical barrier compositions. FIGS. 3A-3E show dissolution kinetics analysis for each of the five samples differing in glycerol content, as follows: 3.125 mL glycerol content (Sample A, FIG. 3A), 6.25 mL glycerol content (Sample B, FIG. 3B), 12.5 mL glycerol content (Sample C, FIG. 3C), 25 mL glycerol content (Sample D, FIG. 3D), and 50 mL glycerol content (Sample E, FIG. 3E). FIG. 3F shows a linear regression analysis of the plot of Sample A (FIG. 3A). FIG. 3G shows a linear regression analysis of the plot of Sample B (FIG. 3B). FIG. 3H shows a linear regression analysis of the plot of Sample C (FIG. 3C). FIG. 3I shows a linear regression analysis of the plot of Sample D (FIG. 3D). FIG. 3J shows a linear regression analysis of the plot of Sample E (FIG. 3E).

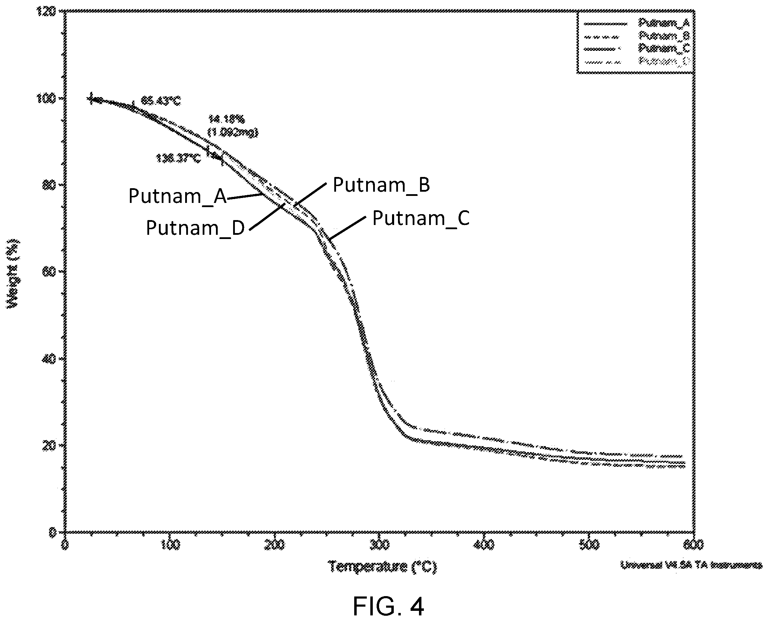

FIG. 4 shows thermogravimetric analysis of a surgical barrier composition that was prepared with water-soluble polysaccharide by weight and glycerol by volume at a ratio of about 1:1, with water present at about 14 wt%. The water-soluble polysaccharide was carboxymethylcellulose sodium salt with MW = 90,500 g/mol. Four samples (Putnam_A, Putnam_B, Putnam_C, Putnam_D) from a single film were measured.

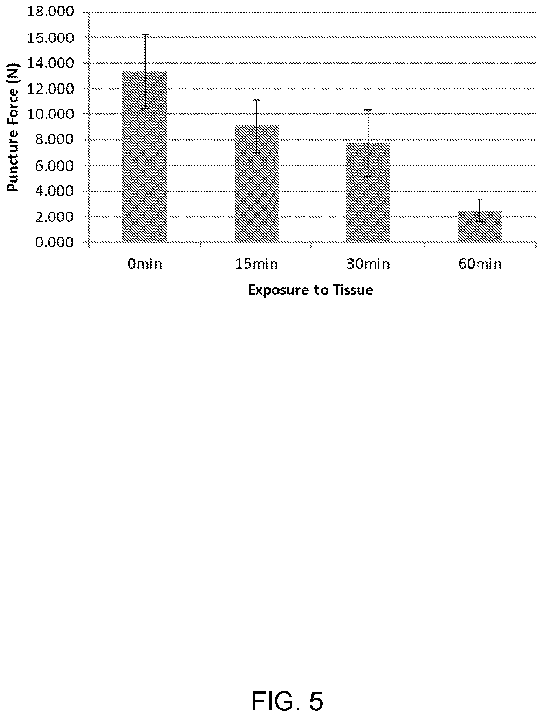

FIG. 5 shows puncture resistance analysis of a surgical barrier composition that was prepared with water-soluble polysaccharide by weight and glycerol by volume at a ratio of about 1:1, with water present at about 14 wt%. The water-soluble polysaccharide was carboxymethylcellulose sodium salt with MW = 90,500 g/mol. The puncture resistance analysis indicated that surgical barrier composition retains substantial needle resistance for at least 30 min.

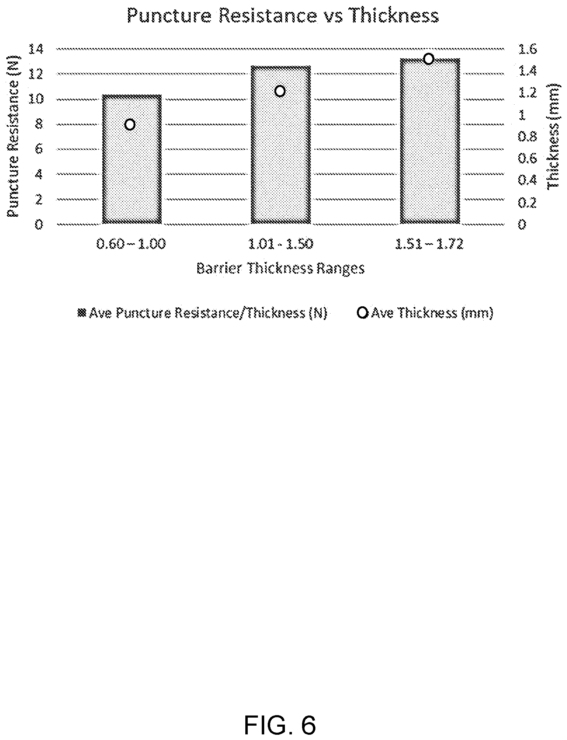

FIG. 6 shows puncture resistance as a function of thickness of a surgical barrier composition that was prepared with water-soluble polysaccharide by weight and glycerol by volume at a ratio of about 1:1, with water present at about 14 wt%. The water-soluble polysaccharide was carboxymethylcellulose sodium salt with MW = 90,500 g/mol. The samples were also analyzed for puncture resistance as a function of thickness. Sample were binned into groups of an average thickness of 0.6-1.00, 1.01-1.50, and 1.51-1.72 mm. The data suggests that for this surgical barrier composition, under these conditions, a thickness of about 1.01-1.50 mm for a surgical barrier would be favorable.

### DETAILED DESCRIPTION OF THE INVENTION

In a first aspect, the present invention is directed to a resorbable surgical barrier sheet comprising:
water-soluble polysaccharide having a molecular weight of 30,000 g/mol to 500,000 g/mol;
glycerol; and
water,
wherein the water-soluble polysaccharide by weight and glycerol by volume are present at a ratio of about 1:0.8 to 1:1.2,
wherein water is present at 8-20 wt%, and
wherein the resorbable surgical barrier sheet possesses a needle penetration resistance of at least 1 Newton and an elastic modulus of 0.5-2 MPa.

In a second aspect, the present invention is directed to a resorbable surgical barrier sheet as defined herein for use in a method of preventing injury to tissue during a surgical procedure, wherein the method comprises placing the resorbable surgical barrier sheet at a surgical site.

Further features of the present invention are as defined in the appended claims.

The present disclosure is directed to a surgical barrier composition which is a solid blend material containing solely or at minimum a homogeneous mixture of a polysaccharide, glycerol, and water. The present disclosure is furthermore directed to a surgical barrier device which contains the surgical barrier composition in whole or in part. Without being bound by theory, the homogeneous mixture is believed to be or include a physical entanglement of the polysaccharide and glycerol. The surgical barrier composition likely includes hydrogen bonding interactions between the polysaccharide, glycerol, and water. In some embodiments, the composition includes a partial level of esterification between the polysaccharide and glycerol, while in other embodiments, the composition substantially or completely excludes esterification.

The water-soluble polysaccharide is at least partially water-soluble (e.g., at least or greater than 70% or 80%), substantially water-soluble (e.g., at least or greater than 90, 95, or 98%) or completely (100%) water-soluble. The polysaccharide may be, for example, a cellulose or hemicellulose, such as a methylcellulose, carboxymethylcellulose (CMC), hydroxyethyl cellulose, hydroxypropyl cellulose, or a salt form (e.g., sodium or ammonium) or ester form thereof (e.g., cellulose acetate, cellulose acetate propionate, or cellulose acetate butyrate), hyaluronic acid (HA), or a starch, or a combination thereof. Polymers of any of the known monosaccharides (e.g., glucose, mannose, xylose, etc.) are considered herein as the polysaccharide, provided they have at least partial or complete solubility in water. In some embodiments, any one or more of any of the above polysaccharides are excluded from the surgical barrier composition.

The at least three components (polysaccharide, glycerol, and water) are present in the surgical barrier composition in any suitable ratio provided that a flexible solid composition with sufficient strength or toughness to resist needle puncture is obtained. In some embodiments, the ratio of polysaccharide, glycerol, and water are expressed in terms of weight, volume, and percent ratio, respectively. In a first set of embodiments, the water-soluble polysaccharide (by weight), glycerol (by volume), and water (as % water) are present in the surgical barrier composition in a ratio of precisely or about 1:0.8:0.8 to 1:1.2:1.2, or within any sub-ratio therein. In a second set of embodiments, the water-soluble polysaccharide (by weight), glycerol (by volume), and water (as % water) are present in the surgical barrier composition in a ratio of precisely or about 1:0.8:0.8 to 1:1.15:1.15. In a third set of embodiments, the water-soluble polysaccharide (by weight), glycerol (by volume), and water (as % water) are present in the surgical barrier composition in a ratio of precisely or about 1:1:1 to 1:1.15:1.15. In a fourth set of embodiments, the water-soluble polysaccharide (by weight), glycerol (by volume), and water (as % water) are present in the surgical barrier composition in a ratio of precisely or about 1:1:1 to 1:1.2:1.2. The water-soluble polysaccharide (by weight), glycerol (by volume), and water (as % water) may alternatively be present in the surgical barrier composition in a more precise ratio of precisely or about, for example, 1:0.8:0.8, 1:0.9:0.9, 1:1:1, 1:1.1:1.1, 1:1.15:1.15, or 1:1.2:1.2, or a range bounded by any two of the foregoing ratios.

In embodiments, the at least three components (polysaccharide, glycerol, and water) of the surgical barrier composition are expressed in terms of a ratio of polysaccharide by weight and glycerol by volume. The water-soluble polysaccharide by weight and glycerol by volume are present in the surgical barrier composition (i.e., solid flexible material) at a ratio of about 1:0.8 to 1:1.2, and any subranges thereof. In embodiments, the water-soluble polysaccharide by weight and glycerol by volume, are present at a ratio of about 1:0.8 to 1:1.15, and any subranges thereof. In embodiments, the water-soluble polysaccharide by weight and glycerol by volume, are present at a ratio of about 1.1 to 1:1.15, and any subranges thereof. In embodiments, the water-soluble polysaccharide by weight and glycerol by volume, are present at a ratio of about 1:1 to 1:1.2, and any subranges thereof. In embodiments, the water present is expressed as a percent of total weight of the surgical barrier composition. The water is present at 8-20 wt%, and any subranges thereof. In embodiments, the water is present at 10-18 wt%, and any subranges thereof. In embodiments, the water is present at 12-16 wt%, and any subranges thereof. In embodiments, the water is present at 14 wt%. In embodiments, the water is present at 8, 9, 10, 11, 12, 13, 14, 15, 16, 17, 18, 19, 20, 21, 22, 23, 24, 25, 26, 27, 28, 29, or 30 wt%.

In embodiments, the water present in the surgical barrier composition is determined by any suitable manner. In some embodiments, the water present is determined by thermogravimetric analysis. In embodiments, the water present is determined by thermogravimetric analysis as measured by any suitable temperature range. In embodiments, the water present is determined by thermogravimetric analysis as measured between 50-300°C, 50-250°C, 50-200°C, or 50-150°C, and any suitable subranges therein. In embodiments, the water present is determined by thermogravimetric analysis as measured between 40-160°C, and any subranges thereof. In embodiments, the water present is determined by thermogravimetric analysis as measured between 60-140°C, and any subranges thereof.

For any of the exemplary ratio values and ranges provided above, the polysaccharide may be selected from one or a combination of any of the polysaccharides disclosed in the present disclosure, such as, for example, a cellulose or hemicellulose, such as a methylcellulose, carboxymethylcellulose (CMC), hydroxyethyl cellulose, hydroxypropyl cellulose, or a salt form (e.g., sodium or ammonium) or ester form thereof (e.g., cellulose acetate, cellulose acetate propionate, or cellulose acetate butyrate), hyaluronic acid (HA), or a starch, or a combination thereof, and including any of the molecular weights or ranges thereof provided in this disclosure.

In a first set of embodiments, in addition to any of the embodiments described above, including any of the polysaccharide compositions and ratios provided above, the polysaccharide has a molecular weight of precisely or about 30,000 g/mol to 500,000 g/mol. In a second set of embodiments, in addition to any of the embodiments described above, including any of the polysaccharide compositions and ratios provided above, the polysaccharide may have a molecular weight of precisely or about 40,000 g/mol to 500,000 g/mol. In a third set of embodiments, in addition to any of the embodiments described above, including any of the polysaccharide compositions and ratios provided above, the polysaccharide may have a molecular weight of precisely or about 30,000 g/mol to 250,000 g/mol. In a fourth set of embodiments, in addition to any of the embodiments described above, including any of the polysaccharide compositions and ratios provided above, the polysaccharide may have a molecular weight of precisely or about 30,000 g/mol to 150,000 g/mol. In a fifth set of embodiments, in addition to any of the embodiments described above, including any of the polysaccharide compositions and ratios provided above, the polysaccharide may have a molecular weight of precisely or about 40,000 g/mol to 150,000 g/mol. In any of the ratio embodiments of the methods described above, the water-soluble polysaccharide (such as any of those provided above) may have a molecular weight of 49,000 g/mol, 90,500 g/mol, or 250,000 g/mol. In any of the ratio embodiments of the methods described above, the water-soluble polysaccharide (such as any of those provided above) may have a molecular weight of 90,500 g/mol.

For any of the ranges in molecular weights provided above, the polysaccharide may be selected from one or a combination of any of the polysaccharides disclosed in the present disclosure, such as, for example, a cellulose or hemicellulose, such as a methylcellulose, carboxymethylcellulose (CMC), hydroxyethyl cellulose, hydroxypropyl cellulose, or a salt form (e.g., sodium or ammonium) or ester form thereof (e.g., cellulose acetate, cellulose acetate propionate, or cellulose acetate butyrate), hyaluronic acid (HA), or a starch, or a combination thereof

The surgical barrier composition possesses a combination of strength (particularly, resistance to needle puncture) and flexibility such that the barrier composition is ideally suited as a protective shield during surgery. For any of the surgical barrier compositions and embodiments described above, the surgical barrier composition typically has an elastic modulus of precisely or about 0.5-2 MPa, for example 0.5-1.5 MPa, 0.5-1 MPa, 0.8-2 MPa, 0.8-1.5 MPa, 0.8-1 MPa, 1-2 MPa, 1-1.5 MPa, 1.25-2 MPa, or 1.5-2 MPa. For any of the surgical barrier composition and embodiments described above, including any of the elastic moduli provided above, the surgical barrier may possess a penetration resistance of at least 1, 2, 3, 4, or 5 Newtons and typically up to 6, 7, 8, 9, 10, 12, or 15 Newtons (e.g., 1-15 N, 2-15 N, 3-15 N, 4-15 N, or 5-15 N). In exemplary embodiments, the surgical barrier material possesses an elastic modulus of precisely or about 0.5-2 MPa or 1-2 MPa and a penetration resistance of precisely or about 1-15 N, 2-15 N, 3-15 N, 4-15 N, or 5-15 N. The properties of the surgical barrier render it capable of shielding biological tissue from inadvertent needle puncture and capable of dissolving at the surgical site after use. In embodiments, the surgical barrier is placed over tissue to create a protective barrier against inadvertent needle puncture during surgery.

In another aspect, the present disclosure is directed to a surgical barrier device at least partially or completely composed of any of the above described surgical barrier compositions or embodiments thereof. The surgical barrier device is typically in the shape of a film or sheet. The film generally has a thickness of, for example, about, precisely, or at least 0.5, 0.6, 0.7, 0.8, 0.9, 1, 2, 3, 4, or 5 mm, or a thickness within a range bounded by any of these two values. For any of the embodiments provided in this disclosure, the film or sheet may have a thickness of at least 0.5 mm and up to 5 mm, or a thickness of at least 1 mm and up to 5 mm, a thickness of at least 1 mm and up to 3 mm, a thickness of at least 0.8 mm and up to 2 mm, a thickness of at least 1 mm and up to 1.5 mm. In different embodiments, including for any of the embodiments already described above, the film may have a thickness of 0.5-5 mm, 0.5-4 mm, 0.5-3 mm, 0.5-2 mm, 0.5-1 mm, 1-5 mm, 1-4 mm, 1-3 mm, 1-2 mm, 2-5 mm, 2-4 mm, 2-3 mm, 3-5 mm, or 4-5 mm. In embodiments, the film has a thickness of 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, or 3.0 mm.

As the surgical barrier film or sheet should be capable of protecting an area of bodily tissue during surgery, the film or sheet will generally have a length-wise diameter (i.e., perpendicular to its thickness) in one or both planar dimensions of at least 1, 2, 3, 4, or 5 centimeters. In some embodiments, the surgical barrier film or sheet has a substantially uniform thickness (e.g., the substantial absence of protrusive or recessive features) while in other embodiments the surgical barrier has a substantially non-uniform thickness (e.g., the presence of protrusive or recessive features, which may help to prevent migration or movement of the surgical barrier during the surgery). The surgical barrier film or sheet may also be non-uniform by being thicker in the middle portion of a sheet of the material while tapering toward the edges of the material. The disclosure also contemplates shapes other than a film, to render the surgical barrier material useful for other or additional purposes, e.g., as a suture (i.e., thread), bandage, band, tube, or sleeve.

In some embodiments, the surgical barrier device further includes at least one component or other feature that prevents migration of the surgical barrier in the body. In some embodiments, the at least one feature that prevents migration includes at least one protrusion, at least one texture, or a combination thereof. The protrusive or recessive feature may be selected from, for example, bumps, dimples, pillars, or a patterned texture. In some embodiments, no metal or plastic component (e.g., clip or other fastening device) is affixed or otherwise attached or bonded to the surgical barrier material, and the surgical barrier material (or entire device) is constructed only of the homogeneous blend of components (polysaccharide, glycerol, and water) described above.

In one embodiment, the film or sheet of the surgical barrier material is a monolith, and thus, not coated or layered with another material. In another embodiment, the film or sheet of the surgical barrier material is coated or layered with another material, in which case the film or sheet can be considered a layer within a multi-layer composite. If another one or more layers are included, the additional layers should also be biocompatible and/or biodegradable (e.g., PLA).

The present application also discloses methods for producing the surgical barrier composition described above. The method generally involves mixing the at least three components (polysaccharide, glycerol, and water), pouring the resultant blend into a mold, and subjecting the mixture to an elevated temperature to evaporate a portion of the water to form a film. In embodiments, the glycerol and water are mixed first, optionally with heating and/or intermittent blending, and the polysaccharide (typically as a solid powdered material) is added with stirring. The resulting mixture is then subjected to an elevated temperature. In embodiments, the elevated temperature is typically at least 40°C, 45°C, or 50°C and up to 55°C, 60°C, 65°C, 70°C, 75°C, or 80°C for a time period of, for example, at least or more than 8, 10, 12, 18, 24, 36, or 48 hours, before removing the finished film from the mold. The foregoing time periods refer to the period of time the mixture of components is heated at any of the temperatures provided above. In some embodiments, a temperature within a range bounded by any two of the exemplary values provided above is used, such as a temperature within a range of, e.g., 40-80°C, 45-80°C, 50-80°C, 55-80°C, 60-80°C, 40-75°C, 45-75°C, 50-75°C, 55-75°C, 60-75°C, 40-70°C, 45-70°C, 50-70°C, 55-70°C, 60-70°C, 40-65°C, 45-65°C, 50-65°C, 55-65°C, 60-65°C, 40-60°C, 45-60°C, 50-60°C, 55-60°C, 40-55°C, 45-55°C, 50-55°C, 40-50°C, or 45-50°C. Similarly, a time period within a range bounded by any two of the exemplary values provided above may be used, such as a time period within a range of, e.g., 8-48 hours, 10-48 hours, 12-48 hours, 18-48 hours, 24-48 hours, 36-48 hours, 8-36 hours, 10-36 hours, 12-36 hours, 18-36 hours, 24-36 hours, 8-24 hours, 10-24 hours, 12-24 hours, or 18-24 hours, for any of the temperatures or range thereof provided above. Any temperature range provided above may be used in combination with any time period provided above, e.g., a temperature of 40-80°C in combination with a time period of 8-48 hours. In embodiments, the methods for producing the barrier composition are adapted for a continuous or automated process by use of industrial equipment and apparatuses adapted for such purpose, as well known in the art.

The present application further discloses a non-claimed method of preventing injury to tissue during a surgical procedure. The method is particularly directed to protecting bodily tissue from needle puncture during surgery. In the method, the surgical barrier device, which may be or include any one of the surgical barrier compositions described above, including any of the embodiments described above, is placed by a user (e.g., a clinician such as a surgeon) at a surgery site on bodily tissue to be protected during surgery. The surgical barrier device used in the method may include any of the polysaccharide compositions provided in this disclosure, any of the ratios and ranges thereof provided in this disclosure, any of the polysaccharide molecular weights and ranges thereof provided in this disclosure, any of the thicknesses and ranges thereof provided in this disclosure, and any of the elastic moduli and/or penetration resistance values provided in this disclosure, and any of these different embodiments may be combined in the surgical barrier device used in the method. Any two or more specific embodiments of the surgical barrier material or device disclosed in the present disclosure may be selected and combined for the method of preventing injury described herein.

At the completion of surgery, the surgical barrier device is left in place through suturing, after which time the surgical barrier device dissolves and is cleared from the body. For any of the surgical barrier embodiments described in this disclosure, the surgical barrier generally exhibits a clearance or degradation profile of at least 90%, 93%, 95%, or 97% clearance or degradation within 3, 4, 5, or 6 hours of contact of the surgical barrier with bodily tissue. For any of the surgical barrier embodiments described in this disclosure, the surgical barrier, after being placed in the surgical site, may have a rate of dissolution in the first 15, 20, 30, or 45 minutes that is slower than the rate of dissolution after the first 15, 20, 30, or 45 minutes, respectively. The surgery may be, for example, abdominal surgery, or more particularly, an abdominal surgery that includes fascial closure or a laparotomy. The surgery may also be other than abdominal surgery, such as heart surgery, coronary artery bypass surgery, tumor removal surgery, or organ transplant or removal surgery.

A significant advantage of the surgical barrier compositions described herein is their ability to simply dissolve at the surgical site with subsequent clearance of the dissolved components through the kidneys or liver, thereby advantageously eliminating the need for their removal after surgery. Indeed, the material can dissolve very rapidly (e.g., within 1-24 hours) and be eliminated from the body. For any of the surgical barrier embodiments described in this disclosure, the surgical barrier material substantially dissolves upon contact with bodily tissue at a surgical site (extracellular fluid) within 72, 48, 36, 24, 20, 18, 15, 12, 10, 7, 5, 3, or 2 hours, or even within 1 hour or 30 minutes. In embodiments, the surgical barrier material or device is reversibly adherable to biological tissue. Because the disclosed surgical barrier material can dissolve at the surgical site, the risks associated with prior known devices are significantly reduced or eliminated. A further advantage of the barrier compositions described herein is biocompatibility of the composition during dissolution and elimination from the body.

There are additional advantages of the described surgical barrier that provide unique clinical and functional attributes. An advantage is that the surgical barrier composition is a film that is configured to resist and/or prevent needle puncture. Another advantage is that the surgical barrier composition is not a paste or powder. Another advantage is that the surgical barrier is placed at the desired site by the user by hand placement, and it does not need to be applied via an applicator device (e.g., a mechanical applicator device). Another advantage is that upon placement on tissue at the desired site, the surgical barrier does not strongly adhere to the tissue upon contact and allows for removal and/or repositioning by the user with no damage to the contacted tissue or minimal damage to the contacted tissue. This advantage of being able to remove and/or reposition the surgical barrier with no or little tissue damage is an advantage over other conventional surgical sheets (e.g., Seprafilm^{™} adhesion barrier) that adhere almost irreversibly upon contact with tissue. Another advantage of the surgical barrier is the attribute of dissolving and being eliminated from the site wherein it is placed within about one day or less of placement by the user.

### EXAMPLES

### Example 1 - Methods of preparing polysaccharide-glycerol penetration-resistant compositions and surgical barriers.

Materials included carboxymethylcellulose (CMC) sodium salt (Ashland, Aqualon CME 7LF PH BET, degree of substitution-0.7; Product code: 891158, Lot # 0551931007, M_{w} = 90,500), glycerol (glycerin) (99.5% anhydrous, USP grade, skincare), and vegetable-based. HUMCO (lot #A44083, exp. 11/2021), and distilled water.

At room temperature, distilled water (212.5 mL) was placed in a borosilicate beaker to which glycerol (12.5 mL) was added via syringe. The combined liquids were mixed at room temperature until a homogeneous solution was reached (~1 minute). The beaker was loosely covered, and the solution placed in an incubator at 50 °C for 1 hour to equilibrate to the same temperature. The beaker was removed from the incubator, and the solution was vigorously stirred at high RPM with an immersion blender. The CMC powder (12.5 g) was added in a steady stream over 15 seconds under vigorous stirring. The suspension was stirred at high speed for 1 minute, then the beaker was placed in the incubator at 50 °C for 1 hour. The CMC/glycerol/water suspension was removed from the incubator, vigorously stirred with the immersion blender for 1 minute and placed back in the incubator at 50 °C for 1 hour (to allow bubbles to come to the surface and clear the solution). After 1 hour, the clear CMC/glycerol/water solution (slightly brownish/yellow) was removed, and 100 mL (two 50 mL syringes full) was placed on a 4"x8" stainless steel tray (2 trays in total). The trays were placed at 50 °C for 24 hours to form the elastomer by evaporation of the water. After 24 hours, the trays were removed from the incubator and the films peeled from the stainless-steel tray surface.

In this experiment, relative humidity was ambient. The effect of relative humidity on film formation and integrity was not determined. Relative humidity may be a controlled manufacturing parameter in some embodiments. In this experiment, a buffered system was not used, although a buffered system may be used in some embodiments. Stirring rotations per minute (RPM) was not controlled in these experiments but may be controlled in some embodiments. The rate of CMC addition to the stirring water/glycerol was not controlled in these experiments but may be controlled in some embodiments.

### Experiment 1

CMC was added to 10% wt/vol (Amazon, food grade, brownish in color). Glycerol was added to 1% (vol/vol). 20 g CMC and 2 mL glycerol were dissolved in 178 ml cold water. Mixture aliquoted into four trays. The mixture was heated in an oven at 60 °C for about 17.5 h. Final water content was 72% on masonry setting. The final film was thin, brittle, and light brown in color.

### Experiment 2

CMC was added to 5% wt/vol (Ashland, Aqualon CME 7LF PH BET, Product code: 891158, Lot # 0551931007, MW = 90,500). Glycerol was added to 2% (vol/vol). 25 g CMC and 10 mL glycerol were dissolved in 465 ml cold water. The mixture was aliquoted into four trays. The mixture was heated in an oven at 50 °C for about 24 h. The final film was strong, flexible, and brownish in color.

### Experiment 3

CMC was added to 5% wt/vol (Ashland, Aqualon CME 7LF PH BET, Product code: 891158, Lot # 0551931007, MW = 90,500). Glycerol was added to 2% vol/vol. 25 g CMC and 10 mL glycerol were dissolved in 465 ml cold water. The mixture did not disperse well in cold water and was stirred by immersion blender for about 5 min. The mixture was placed at 50°C for about 2 h after which bubbles disappeared but a thick viscous sediment was present at the bottom of the mixing container. The mixture was stirred by immersion blender for about 2 min and returned to 50°C for about 1 h. After a second heating, the mixture became clear and lightly viscous. The mixture was aliquoted into four trays. The mixture was heated in a VWR gravity convection oven at 50 °C for about 24 h after which the samples were thick and viscous and not yet solidified. The samples were heated for an additional 12 hours at 62°C. The final film was thin and brittle and could not be removed from the trays.

### Experiment 4

CMC was added to 5% wt/vol (Ashland, Aqualon CME 7LF PH BET, Product code: 891158, Lot # 0551931007, MW = 90,500). Glycerol was added to 10% vol/vol. 50 mL glycerol was dissolved in 425 ml cold water and the mixture heated at 50°C for 1 hour. 25 g of powder CMC was then added while mixing with immersion blender. The mixture was placed at 50°C for 30 min and was stirred by immersion blender. The mixture was placed at 50°C for 30 min after which bubbles disappeared. The mixture was aliquoted into four trays. The samples were heated at 50°C for 24 h. The final film was clear and strong, but broke when forcefully pulled on.

### Experiment 5

CMC was added to 5% wt/vol (Ashland, Aqualon CME 7LF PH BET, MW = 49,000). Glycerol was added to 10% vol/vol. 50 mL glycerol was dissolved in 425 ml cold water and the mixture was heated at 50°C for 1 hour. 25 g of powder CMC was then added while mixing with the immersion blender. The mixture was placed at 50°C for 30 min and was stirred by immersion blender. The mixture was placed at 50°C for 30 min after which bubbles had disappeared. The mixture was aliquoted into four trays. The samples were heated at 50°C for 24 h. The final film was clear and strong, but broke when forcefully pulled on.

### Experiment 6

CMC was added to 5% wt/vol (Ashland, Aqualon CME 7LF PH BET, MW = 250,000). Glycerol was added to 10% vol/vol. 50 mL glycerol was dissolved in 425 ml cold water and the mixture heated at 50°C for 24 h. 25 g of powder CMC was then added while mixing with the immersion blender. The mixture placed at 50°C for about 7.5 h. The mixture was thick with bubbles. The mixture was aliquoted into two trays. The samples were heated at 50°C for 24 h. The final film was clear and strong, but broke when forcefully pulled on.

### Experiment 7

CMC was added to 5.4% wt/vol (Ashland, Aqualon CME 7LF PH BET, MW = 250,000). Glycerol was added to 2.7% vol/vol. 25 mL glycerol was dissolved in 425 ml cold water and the mixture heated at 50°C for 1 h. 25 g of powder CMC was then added while mixing with the immersion blender. The mixture was placed at 50°C for about 8 h. The mixture was clear with some small bubbles. 200 mL of the mixture was aliquoted into each of two trays. The samples were heated at 50°C for 24 h.

### Experiment 8

CMC was added to 5.26% wt/vol (Ashland, Aqualon CME 7LF PH BET, MW = 90,500). Glycerol was added to 5.26% vol/vol. 12.5 mL glycerol was dissolved in 212.5 mL cold water and the mixture heated at 50°C for 1 h. 12.5 g of powder CMC was then added while mixing with the immersion blender. 200 mL of the mixture was aliquoted into each of two trays. The samples were heated at 50°C for 24 h. The final film was clear and strong.

Table 1 below shows preparations of compositions from Experiments 1-8 prepared at the indicated component concentrations and conditions.

**Table 1**

| Experiment No. | % CMC | % Glycerol | CMC MW |
|---|---|---|---|
| 1 | 10 | 1 | Food grade |
| 2 | 5 | 2 | 90,500 |
| 3 | 5 | 2 | 90,500 |
| 4 | 5 | 10 | 90,500 |
| 5 | 5 | 10 | 49,000 |
| 6 | 5 | 10 | 250,000 |
| 7 | 5.4 | 2.7 | 250,000 |
| 8 | 5.26 | 5.26 | 90,500 |

### Example 2 - Materials characterization of polysaccharide-glycerol penetration-resistant compositions

Five different compositions of the product were fabricated to contain a constant weight of carboxymethylcellulose (25 g) with varying amounts of glycerol (3.125 mL, 6.25 mL, 12.5 mL, 25 mL or 50 mL) in the starting mixtures. The starting mixtures were further processed and then characterized. The materials characterization included three sets of studies to determine the effect of glycerol content on the materials, as follows: 1) thermogravimetric analysis to measure residual water content, 2) dynamic mechanical analysis to measure the elastic modulus at room temperature (23 °C) and body temperature (37 °C), 3) dissolution kinetics to determine the pattern of material dissolution, and to measure the dissolution half-lives.

Materials. Carboxymethylcellulose (Ashland, Aqualon^{™}, weight average molecular weight 49,000, 0.7 degree of substitution, product number CMC 7L2P BET), abbreviated "CMC". Glycerol, USP. Deionized water.

### Fabrication methods.

At room temperature, distilled water (400 mL) was placed in a 1-liter beaker to which glycerol (3.125 mL (Sample A), 6.25 mL (Sample B), 12.5 mL (Sample C), 25 mL (Sample D) or 50 mL (Sample E)) was added via syringe. The combined liquids were mixed at room temperature until a homogeneous solution was reached (~1 minute). The beaker was loosely covered, and the solution placed in an incubator at 60 °C for 1 hour to equilibrate to the same temperature. The beaker was removed from the incubator, and the solution was vigorously stirred at high RPM. CMC powder (25 g) was added in a steady stream over 15 seconds under vigorous stirring. The suspension was stirred at high speed for 1 minute, then the beaker was placed in the incubator at 60 °C for 1 hour. The CMC/glycerol/water mixture was removed from the incubator, vigorously stirred with the immersion blender for 1 minute, and placed back in the incubator at 60 °C for 1 hour. After 1 hour, the CMC/glycerol/water solution (clear and slightly brownish/yellow) was removed from the incubator and cast onto a single 4"x8" and level tray. The tray was placed at 60 °C with ~90% relative humidity for 48 hours to form the elastomer by evaporation of the water. After 48 hours, the trays were removed from the incubator and the films peeled from the tray surface and sealed in a vapor proof container.

### Thermogravimetric analysis

The five resulting films were subjected to gravimetric analysis to measure residual water content. A TGA Q500 V6.7 instrument was used. FIG. 1A-1E show residual water content for each of the following samples: 3.125 mL glycerol content (Sample A, FIG. 1A), 6.25 mL glycerol content (Sample B, FIG. 1B), 12.5 mL glycerol content (Sample C, FIG. 1C), 25 mL glycerol content (Sample D, FIG. 1D) or 50 mL glycerol content (Sample E, FIG. 1E). The residual water content is plotted against glycerol content in FIG. 1F. The analysis indicates that the residual water content correlates approximately linearly with glycerol content.

### Dynamic mechanical analysis

The five resulting films were subject to dynamic mechanical analysis to determine the effect of glycerol content on elastic modulus. A TA Instruments DMA Q800 Dynamic Mechanical Thermal Analyzer was used to perform the analysis. FIGS. 2A and 2B show elastic modulus as a function of glycerol content at a temperature of 23°C and 37°C, respectively, for the five samples differing in glycerol content. FIGS. 2C-2G show stress-strain curves for each of the following samples: 3.125 mL glycerol content (Sample A, FIG. 2C), 6.25 mL glycerol content (Sample B, FIG. 2D), 12.5 mL glycerol content (Sample C, FIG. 2E), 25 mL glycerol content (Sample D, FIG. 2F) or 50 mL glycerol content (Sample E, FIG. 2G). The analysis indicates that: 1) Elastic modulus scales non-linearly with glycerol content; and 2) the elastic modulus is influenced non-linearly by temperature, but decreases in all samples with temperature change from 23 °C to 37 °C.

### Dissolution kinetics analysis

The five resulting films were subjected to dissolution kinetics analysis to determine the influence of glycerol content on the rate of film dissolution in water at 37 °C. An analytical balance, glass vials, 37 °C incubator with agitation, and 60 °C incubator/vacuum to dry samples were used for the analysis. FIGS. 3A-3E show dissolution kinetics analysis for each of the five samples differing in glycerol content, as follows: 3.125 mL glycerol content (Sample A, FIG. 3A), 6.25 mL glycerol content (Sample B, FIG. 3B), 12.5 mL glycerol content (Sample C, FIG. 3C), 25 mL glycerol content (Sample D, FIG. 3D), and 50 mL glycerol content (Sample E, FIG. 3E). FIG. 3F shows a linear regression analysis of the plot of Sample A (FIG. 3A). FIG. 3G shows a linear regression analysis of the plot of Sample B (FIG. 3B). FIG. 3H shows a linear regression analysis of the plot of Sample C (FIG. 3C). FIG. 3I shows a linear regression analysis of the plot of Sample D (FIG. 3D). FIG. 3J shows a linear regression analysis of the plot of Sample E (FIG. 3E). The analysis indicates that: 1) there is an about 30-minute lag before material loss due to dissolution; and 2) the dissolution half-life of the materials under infinite sink conditions scales non-linearly with glycerol content.

### Example 3 - Methods of preparing polysaccharide-glycerol penetration-resistant compositions and surgical barriers.

Materials: Carboxymethylcellulose sodium salt (Ashland, Aqualon CME 7LF PH BET, degree of substitution-0.7; Product code: 891158, Lot # 0551931007, Mw = 90,500 g/mol). Glycerol (glycerin) (99.5% anhydrous, USP grade, skincare). Vegetable-based. HUMCO (lot #A44083, exp. 11/2021). Distilled water

### Method of preparation.

A surgical barrier was prepared with water-soluble polysaccharide by weight and glycerol by volume at a ratio of about 1:1, with water present at about 14 wt%. The water-soluble polysaccharide was carboxymethylcellulose sodium salt with MW = 90,500 g/mol. At room temperature, distilled water (425 mL) was placed in a 1-liter beaker to which glycerol (25 mL) was added via syringe. The combined liquids were mixed at room temperature until a homogeneous solution was reached (~1 minute). The beaker was loosely covered, and the solution was placed in an incubator at 60 °C for 1 hour to equilibrate to the same temperature. The beaker was removed from the incubator, and the solution was vigorously stirred at high RPM with an immersion blender. The CMC powder (25 g) was added in a steady stream over 15 seconds under vigorous stirring. The suspension was stirred at high speed for 1 minute, then the beaker was placed in the incubator at 60 °C for 1 hour. The CMC/glycerol/water suspension was removed from the incubator, vigorously stirred with the immersion blender for 1 minute and placed back in the incubator at 60 °C for 1 hour (to allow bubbles to come to the surface and clear the solution). After 1 hour, the clear CMC/glycerol/water solution (slightly brownish/yellow) was removed, and 200 mL (four 50 mL syringes full) was placed on a 4"x8" stainless steel tray (2 trays in total). The trays were placed at 60 °C at 90% relative humidity for 36 hours to form the elastomer by evaporation of the water. After 36 hours, the trays were removed from the incubator and the films peeled from the stainless-steel tray surface.

### Residual Water Content Measurement.

Residual water was measured using a TA Instruments Q500 Thermogravimetric Analyzer. Four samples (Putnam_A, Putnam_B, Putnam_C, Putnam_D) from a single film were measured. FIG. 4 shows the results for each of the four samples. The results show that water was tightly bound to the film matrix with no sharp water loss at 100 °C. The slow, gradual water loss between 65 °C and 136 °C was indicative of water evaporation from a highly hydrophilic matrix to which the water was strongly bound by hydrogen binding. These results were consistent with the composition of the surgical barrier. The results were consistent among the four samples analyzed with all four surgical barriers containing ~14% water.

### Example 4

### Preparation of compositions

Polysaccharide-glycerol penetration-resistant compositions were prepared as generally described in Example 3, but with varying amounts of glycerol. Briefly, five compositions were prepared with either 3 ml, 6 ml, 12 ml, 25 ml, or 50 ml of glycerol. 25 g of carboxymethylcellulose sodium salt (MW=90,500) was used in each of the samples. Table 2 below shows the amount each of carboxymethylcellulose and glycerol in each composition and the ratio of carboxymethylcellulose by weight to glycerol by volume.

**Table 2.**

| *Composition* | *Carboxymethylcellulose (g)* | *Glycerol (ml)* | *Ratio CMC:glycerol* |
|---|---|---|---|
| 1 | 25 | 3 | 1:0.12 |
| 2 | 25 | 6 | 1:0.24 |
| 3 | 25 | 12 | 1:0.48 |
| 4 | 25 | 25 | 1:1 |
| 5 | 25 | 50 | 1:2 |

### Compositions 1-5 were formed into films are described in Example 3.

### Puncture resistance analysis

Puncture resistance analysis was carried out for the surgical barriers comprising Compositions 1-5. The puncture resistance analysis comprised destructive testing based on ASTM F1342 and F2878. The puncture resistance analysis comprised cut samples of the surgical barriers punctured with a straightened surgical needle. The samples were supported in a clamping fixture on the upper crosshead while the needle was held in a lower vice-grip on the load-cell of a hydraulic-testing machine. Baseline puncture resistance was first measured for the samples without exposure to tissue. Subsequent measurements were made on samples that were contacted with tissue. The hydraulic-testing machine drove the needle into the respective sample at a predetermine displacement at a constant rate which ensured full penetration of the samples.

Materials: Hydraulic-testing machine (Test Resources Nano (Model SS 2370) with 51b (22N) Load Cell (Test Resources WF-5)); Surgical Film Barrier samples (cut into 2 cm x 2 cm samples); GS24 (40mm) taper needle - straightened; Custom upper grip to hold fixture; and Lower vice-grip with wavey jaws to grasp needle.

### Methods:

Samples (2 cm x 2 cm) were prepared from each of Composition 1(Glycerol 3.125 ml), Composition 2(Glycerol 6.25 ml), Composition 3(Glycerol 12.5ml), Composition 4(Glycerol 25ml), and Composition 5(Glycerol 50ml). A first group of samples were analyzed without exposure to tissue. A second group of samples were analyzed after contacting with tissue. The group of samples that were contacted with tissue were contacted with moistened abdominal rectus from swine (fascial side facing the film) for the time lengths as indicated. At the end of the noted contact time with tissue, the sample was removed from the tissue and analyzed. Each group of samples was tested in triplicate.

### Steps:

1. Prepare samples. Six samples (3 that were not exposed to tissue, 3 that were contacted with tissue) per time point for each composition were prepared. The samples corresponding to contact with tissue were contacted with swine tissue (fascia side to specimen) for the respective time length.
2. Mount samples. Individual samples were centered over an individual 10 mm hole in the testing fixture. The fixture was secured and mounted to upper crosshead.
3. Probe was positioned with ~16 mm clearance from bottom of fixture to allow rotation and removal of fixture.
4. The system was calibrated to 0 N and 0 mm.
5. Needle was displaced at a rate of 50.8 cm/min through 36 mm and returned to original position obtain measurement A.
6. Fixture was rotated 120 degrees and step 4 was repeated to obtain measurement B.
7. Steps 5-6 were repeated to obtain measurement C.

### Thickness analysis.

Where possible, the samples were measured for thickness at various time points before and after contact with tissue (0 min, 15 min, 30 min). Thickness measurements were taken for the 0 min timepoint. However, it was not possible to obtain thickness measurements at all time points. For example, the Composition 5 (Glycerol 50 mL) samples at either 15 or 30 min were not able to be measured as the samples became overly pliable. Also, the Composition 4 (Glycerol 25 mL) samples at 30 min were not able to be measured as a result of the film becoming overly pliable. The Composition 3 (Glycerol 12.5 mL) samples retained some of the fascia tissue as they were removed making thickness evaluation impossible for both time points. Sample thickness is reported in Table 3.

**Table 3. Thickness (mm)**

| Sample | Time (min) | Measurement | | |
|---|---|---|---|---|
| | | A (mm) | B (mm) | C (mm) |
| Composition 1(Glycerol 3.125 ml) | 0 | 0.49 | 0.48 | |
| Composition 2(Glycerol 6.25 ml) | 0 | 0.67 | 0.64 | 0.57 |
| Composition 3(Glycerol 12.5ml) | 0 | 0.70 | 0.72 | 0.72 |
| Composition 4(Glycerol 25ml) | 0 | 1.26 | 1.27 | 1.25 |
| Composition 4(Glycerol 25ml) | 15 | 1.42 | 1.46 | 1.48 |
| Composition 5(Glycerol 50ml) | 0 | 1.72 | 1.75 | 1.75 |

Table 4 shows results for puncture resistance analysis for Compositions 1-5. Analysis was carried out as described above.

**Table 4.**

| Sample | Time (min) | Measurement | | | Average | Std Dev |
|---|---|---|---|---|---|---|
| | | A (N) | B (N) | C (N) | | |
| Composition 1(Glycerol 3.125 ml) | 0 | 24.94 | 25.60 | - | 25.27 | 0.47 |
| Composition 2(Glycerol 6.25 ml) | 0 | 16.92 | 15.72 | 16.24 | 16.29 | 0.60 |
| Composition 3(Glycerol 12.5ml) | 0 | 9.51 | 13.19 | 9.82 | 10.84 | 2.04 |
| Composition 3(Glycerol 12.5ml) | 15 | 1.08 | 0.28 | 0.19 | 0.51 | 0.49 |
| Composition 3(Glycerol 12.5ml) | 30 | 0.63 | 0.80 | 0.21 | 0.54 | 0.30 |
| Composition 4(Glycerol 25ml) | 0 | 6.20 | 5.97 | 5.88 | 6.02 | 0.16 |
| Composition 4(Glycerol 25ml) | 15 | 4.57 | 2.78 | 3.03 | 3.46 | 0.97 |
| Composition 4(Glycerol 25ml) | 30 | 0.11 | 3.31 | 2.89 | 2.10 | 1.74 |
| Composition 5(Glycerol 50ml) | 0 | 0.89 | 1.07 | 1.01 | 0.99 | 0.09 |
| Composition 5(Glycerol 50ml) | 15 | 0.53 | 0.40 | 0.58 | 0.51 | 0.09 |
| Composition 5(Glycerol 50ml) | 30 | 0.21 | 0.39 | 0.25 | 0.28 | 0.09 |

Compositions 1-2 were puncture resistance analyzed at 0 min (no exposure to tissue). Because of high load readings, a measurement C could not be obtained for Composition 1 (Glycerol 3.125 mL). The maximum load was obtained from the load-position curve of each sample and reported above in Table 4. The variability in the maximum load generally increased with increased exposure to the tissue. It was noted that the maximum load for several Measurement A of the Composition 4 (Glycerol 25 mL) group at 30 min of tissue exposure exhibited a different load profile compared to Measurements B and C (See Table 4). While care was taken removing the samples after contact with tissue, it is possible that damage to the samples occurred. The puncture resistance analysis indicated that the samples possessed differing puncture resistance based on the ratio of polysaccharide to glycerol. Composition 4 (Glycerol 25 mL) has favorable properties related to puncture resistance. Additionally, Composition 4 (Glycerol 25 mL) has favorable properties related to puncture resistance after contact with tissue.

### Puncture resistance analysis of Composition 4 (Glycerol 25 mL)

Samples of Composition 4 (Glycerol 25 mL) were prepared as described above. The samples were between 1.5-1.7 mm. The samples were contacted with tissue for 0, 15, 30, and 60 min. The samples were then analyzed for puncture resistance as described above. The results are shown in FIG. 5. The puncture resistance analysis indicated that Composition 4 (Glycerol 25 mL) retains substantial needle resistance for at least 30 min.

The samples were also analyzed for puncture resistance as a function of thickness. Sample were binned into groups of an average thickness of 0.6-1.00, 1.01-1.50, and 1.51-1.72 mm. The results of puncture resistance as a function of thickness are shown in Table 5. The results are also shown in FIG.6.

**Table 5.**

| Average Thickness (mm) | Average Puncture Resistance/Thickness (N) | Average Thickness (mm) | Increase in Resistance | Increase in Thickness |
|---|---|---|---|---|
| 0.60 - 1.00 | 10.3300 | 0.89 | | |
| 1.01 - 1.50 | 12.6600 | 1.22 | 23% | 37% |
| 1.51 - 1.72 | 13.2200 | 1.51 | 4% | 24% |

The results indicate that puncture resistance increases with increasing thickness. An increase in the average puncture resistance to thickness is shown as thickness increases from 0.6-1.00 mm to 1.01-1.50 mm. Likewise, an increase in the average puncture resistance to thickness is shown as thickness increases from 1.01-1.50 mm to 1.51-1.72 mm but the increase is less than the 0.6-1.00 mm to 1.01-1.50 mm comparison. The data suggests that for Composition 4 (Glycerol 25 mL), under these conditions, a thickness of 1.01-1.50 mm for a surgical barrier would be favorable.

## Claims

1. A resorbable surgical barrier sheet comprising:
water-soluble polysaccharide having a molecular weight of 30,000 g/mol to 500,000 g/mol;
glycerol; and
water,
wherein the water-soluble polysaccharide by weight and glycerol by volume are present at a ratio of about 1:0.8 to 1:1.2,
wherein water is present at 8-20 wt%, and
wherein the resorbable surgical barrier sheet possesses a needle penetration resistance of at least 1 Newton and an elastic modulus of 0.5-2 MPa.

2. The resorbable surgical barrier sheet of claim 1, wherein said water-soluble polysaccharide is a cellulose, optionally wherein the cellulose is methyl cellulose, carboxymethylcellulose or a salt thereof (CMC), hyaluronic acid (HA) or a combination thereof.

3. The resorbable surgical barrier sheet according to either of claim 1 or claim 2, wherein the water-soluble polysaccharide by weight and glycerol by volume are present at a ratio of about 1:0.8 to 1:1.15, about 1:1 to 1:1.15, or about 1:1 to 1:1.2.

4. The resorbable surgical barrier sheet according to any one of the preceding claims, wherein water is present at 10-18 wt%, 12-16 wt%, or about 14 wt%.

5. The resorbable surgical barrier sheet according to any one of the preceding claims, wherein water present is determined by thermogravimetric analysis, optionally as measured between 60-140°C.

6. The resorbable surgical barrier sheet according to any one of the preceding claims, wherein the water-soluble polysaccharide has a molecular weight of 40,000 g/mol to 500,000 g/mol, 30,000 g/mol to 250,000 g/mol, 30,000 g/mol to 150,000 g/mol, 40,000 g/mol to 150,000 g/mol, or about 49,000 g/mol, 90,500 g/mol, or 250,000 g/mol.

7. The resorbable surgical barrier sheet according to any one of the preceding claims, wherein the resorbable surgical barrier sheet has a thickness of at least 0.5 mm and up to 5 mm, at least 1 mm and up to 5 mm, at least 1 mm and up to 3 mm, at least 0.8 mm and up to 2 mm, or at least 1.0 mm and up to 1.5 mm.

8. The resorbable surgical barrier sheet according to any one of the preceding claims, wherein the resorbable surgical barrier sheet has a non-uniform thickness or a uniform thickness.

9. The resorbable surgical barrier sheet according to any one of the preceding claims, further comprising at least one feature that prevents migration, wherein optionally the at least one feature that prevents migration comprises at least one protrusion, at least one texture, or a combination thereof.

10. The resorbable surgical barrier sheet according to any one of the preceding claims, wherein the resorbable surgical barrier sheet is reversibly adherable to biological tissue.

11. A resorbable surgical barrier sheet as defined in any of claims 1-10 for use in a method of preventing injury to tissue during a surgical procedure, wherein the method comprises placing the resorbable surgical barrier sheet at a surgical site.

12. The resorbable surgical barrier sheet for use according to claim 11, wherein the resorbable surgical barrier sheet has a needle penetration resistance between 1 Newton and 10 Newton.

## Patentansprüche

1. Resorbierbare chirurgische Barrierefolie, die Folgendes umfasst:
wasserlösliches Polysaccharid mit einem Molekulargewicht von 30.000 g/mol bis 500.000 g/mol;
Glycerin; und
Wasser,
wobei das wasserlösliche Polysaccharid nach Gewicht und das Glycerin nach Volumen in einem Verhältnis von etwa 1:0,8 bis 1:1,2 vorliegen,
wobei Wasser zu 8-20 Gew.-% vorliegt und
wobei die resorbierbare chirurgische Barrierefolie eine Nadelpenetrationsfestigkeit von mindestens 1 Newton und einen Elastizitätsmodul von 0,5-2 MPa aufweist.

2. Resorbierbare chirurgische Barrierefolie nach Anspruch 1, wobei das genannte wasserlösliche Polysaccharid eine Cellulose ist, wobei die Cellulose optional Methylcellulose, Carboxymethylcellulose oder ein Salz davon (CMC), Hyaluronsäure (HA) oder eine Kombination davon ist.

3. Resorbierbare chirurgische Barrierefolie nach Anspruch 1 oder Anspruch 2, wobei das wasserlösliche Polysaccharid nach Gewicht und Glycerin nach Volumen in einem Verhältnis von etwa 1:0,8 bis 1:1,15, etwa 1:1 bis 1:1,15 oder etwa 1:1 bis 1:1,2 vorliegen.

4. Resorbierbare chirurgische Barrierefolie nach einem der vorherigen Ansprüche, wobei Wasser zu 10-18 Gew.-%, 12-16 Gew.-% oder etwa 14 Gew.-% vorhanden ist.

5. Resorbierbare chirurgische Barrierefolie nach einem der vorherigen Ansprüche, wobei der Wassergehalt durch thermogravimetrische Analyse, optional gemessen zwischen 60 und 140 °C, bestimmt wird.

6. Resorbierbare chirurgische Barrierefolie nach einem der vorherigen Ansprüche, wobei das wasserlösliche Polysaccharid ein Molekulargewicht von 40.000 g/mol bis 500.000 g/mol, 30.000 g/mol bis 250.000 g/mol, 30.000 g/mol bis 150.000 g/mol, 40.000 g/mol bis 150.000 g/mol oder etwa 49.000 g/mol, 90.500 g/mol oder 250.000 g/mol aufweist.

7. Resorbierbare chirurgische Barrierefolie nach einem der vorherigen Ansprüche, wobei die resorbierbare chirurgische Barrierefolie eine Dicke von mindestens 0,5 mm und bis zu 5 mm, mindestens 1 mm und bis zu 5 mm, mindestens 1 mm und bis zu 3 mm, mindestens 0,8 mm und bis zu 2 mm oder mindestens 1,0 mm und bis zu 1,5 mm aufweist.

8. Resorbierbare chirurgische Barrierefolie nach einem der vorherigen Ansprüche, wobei die resorbierbare chirurgische Barrierefolie eine ungleichmäßige Dicke oder eine gleichmäßige Dicke aufweist.

9. Resorbierbare chirurgische Barrierefolie nach einem der vorherigen Ansprüche, die ferner mindestens ein Merkmal umfasst, das Migration verhindert, wobei das mindestens eine Migration verhindernde Merkmal optional mindestens einen Vorsprung, mindestens eine Struktur oder eine Kombination davon umfasst.

10. Resorbierbare chirurgische Barrierefolie nach einem der vorherigen Ansprüche, wobei die resorbierbare chirurgische Barrierefolie reversibel an biologischem Gewebe haften kann.

11. Resorbierbare chirurgische Barrierefolie nach einem der Ansprüche 1-10 zur Verwendung in einem Verfahren zum Verhindern von Gewebeverletzungen während eines chirurgischen Eingriffs, wobei das Verfahren das Platzieren der resorbierbaren chirurgischen Barrierefolie an einer Operationsstelle beinhaltet.

12. Resorbierbare chirurgische Barrierefolie zur Verwendung nach Anspruch 11, wobei die resorbierbare chirurgische Barrierefolie eine Nadelpenetrationsfestigkeit zwischen 1 Newton und 10 Newton aufweist.

## Revendications

1. Membrane de barrière chirurgicale résorbable comprenant :
un polysaccharide soluble dans l'eau ayant un poids moléculaire de 30.000 g/mol à 500.000 g/mol ;
du glycérol ; et
de l'eau,
dans laquelle le polysaccharide soluble dans l'eau en poids et le glycérol en volume sont présents à un rapport d'environ 1:0,8 à 1:1,2,
dans laquelle l'eau est présente à 8-20 % en poids, et
où la membrane de barrière chirurgicale résorbable possède une résistance à la pénétration d'aiguille d'au moins 1 newton et un module d'élasticité de 0,5-2 MPa.

2. Membrane de barrière chirurgicale résorbable selon la revendication 1, dans laquelle ledit polysaccharide soluble dans l'eau est une cellulose, optionnellement dans laquelle la cellulose est de la méthylcellulose, carboxyméthylcellulose ou un sel de celle-ci (CMC), de l'acide hyaluronique (HA) ou une combinaison de ceux-ci.

3. Membrane de barrière chirurgicale résorbable selon la revendication 1 ou la revendication 2, dans laquelle le polysaccharide soluble dans l'eau en poids et le glycérol en volume sont présents à un rapport d'environ 1:0,8 à 1:1,15, d'environ 1:1 à 1:1,15 ou d'environ 1:1 à 1:1,2.

4. Membrane de barrière chirurgicale résorbable selon l'une quelconque des revendications précédentes, dans laquelle l'eau est présente à 10-18 % en poids, à 12-16 % en poids ou à environ 14 % en poids.

5. Membrane de barrière chirurgicale résorbable selon l'une quelconque des revendications précédentes, dans laquelle l'eau présente est déterminée par analyse thermogravimétrique, optionnellement comme mesurée entre 60-140°C.

6. Membrane de barrière chirurgicale résorbable selon l'une quelconque des revendications précédentes, dans laquelle le polysaccharide soluble dans l'eau a un poids moléculaire de 40.000 g/mol à 500.000 g/mol, de 30.000 g/mol à 250.000 g/mol, de 30.000 g/mol à 150.000 g/mol, de 40.000 g/mol à 150.000 g/mol, ou d'environ 49.000 g/ml, 90.500 g/mol ou 250.000 g/mol.

7. Membrane de barrière chirurgicale résorbable selon l'une quelconque des revendications précédentes, où la membrane de barrière chirurgicale résorbable a une épaisseur d'au moins 0,5 mm et de jusqu'à 5 mm, d'au moins 1 mm et de jusqu'à 5 mm, d'au moins 1 mm et de jusqu'à 3 mm, d'au moins 0,8 mm et de jusqu'à 2 mm ou d'au moins 1,0 mm et de jusqu'à 1,5 mm.

8. Membrane de barrière chirurgicale résorbable selon l'une quelconque des revendications précédentes, où la membrane de barrière chirurgicale résorbable a une épaisseur non uniforme ou une épaisseur uniforme.

9. Membrane de barrière chirurgicale résorbable selon l'une quelconque des revendications précédentes, comprenant en outre une caractéristique qui empêche la migration, dans laquelle optionnellement la au moins une caractéristique qui empêche la migration comprend au moins une saillie, au moins une texture ou une combinaison de celles-ci.

10. Membrane de barrière chirurgicale résorbable selon l'une quelconque des revendications précédentes, où la membrane de barrière chirurgicale résorbable peut adhérer de manière réversible à un tissu biologique.

11. Membrane de barrière chirurgicale résorbable selon l'une quelconque des revendications 1-10 pour utilisation dans une méthode de prévention de lésion à un tissu pendant une procédure chirurgicale, où la méthode comprend placer la membrane de barrière chirurgicale résorbable à un site chirurgical.

12. Membrane de barrière chirurgicale résorbable selon la revendication 11, où la membrane de barrière chirurgicale résorbable a une résistance à la pénétration d'aiguille d'entre 1 newton et 10 newtons.
